# EUROPEAN PATENT SPECIFICATION

(11) **EP 1 830 802 B1**
(45) Date of publication and mention of the grant of the patent: **04.07.2012**
(21) Application number: 05810004.1
(22) Date of filing: 12.10.2005
(51) Int. Cl.: A61K 9/46, A23G 4/06, A61K 9/00, A23G 4/02, A61Q 11/00

(54) **EFFERVESCENT PRESSED CHEWING GUM TABLET COMPOSITIONS**
SCHÄUMENDE GEPRESSTE KAUGUMMI-TABLETTENZUSAMMENSETZUNGEN
COMPOSITIONS EFFERVESCENTES DE COMPRIMES DE GOMME À MÂCHER

(30) Priority: 13.10.2004 US 618222 P
(43) Date of publication of application: 12.09.2007
(73) Proprietor: Kraft Foods Global Brands LLC, Northfield, IL 60093 (US)
(72) Inventor: GEBRESELASSIE, Petros, Highland Park NJ 08904 (US); BOGHANI, Navroz, Flanders, NJ 07836 (US)
(74) Representative: Wilson Gunn
(86) International application number: PCT/US2005/036929
(87) International publication number: WO 2006/044595

(56) References cited:
- EP-A- 0 424 706
- WO-A-00/35418
- US-A1- 2001 006 677

## Description

### FIELD

The present invention includes pressed gum tablets that effervesce when chewed. The pressed gum tablets may deliver active components to the oral cavity, such as flavors and oral care actives. In particular, the effervescent pressed gum tablets may include a particulate chewing gum base, a tableting powder and an effervescent system.

### BACKGROUND

Effervescent consumable products are desirable because they assist in the delivery of active components into the oral cavity. In particular, effervescence produced during chewing may dilate the oral membranes of the user. Actives having low water solubility may penetrate the dilated oral membranes more easily than in the absence of such effervescence. As such, the bioavailability of the active may be improved.

One of the obstacles facing effervescent consumable products, however, is shelf life. In particular, the edible acid and base used to produce effervescence need to be kept separate until effervescence is desired: Small levels of moisture found in consumable products, such as conventional molten chewing gums, may lead to premature reaction of the acid and base and therefore premature effervescence. In addition, molten gum bases tend to coat the materials and thereby reduce the reaction between edible acids and bases needed to generate effervescence.

U.S. Patent No. 6,235,318 to Lombardy, Jr. et al. (hereinafter "Lombardy") attempts to overcome such problems associated with conventional chewing gums by separating the acid and base components. In particular, Lombardy incorporates the base into the molten chewing gum base and separates the acid into a coating that encapsulates the gum base. Mastication initially causes the coating to dissolve, thereby releasing the acid. Upon further mastication, the acid and base will react. Because the chewing gums of Lombardy are conventional molten chewing gums, they contain a level of moisture that will allow premature reaction between the acid and base if not separated into the gum base and coating.

The present invention, rather, provides a chewing gum product that is generally dry and thus does not lead to premature reaction between the acid and base. In particular, the present invention provides pressed gum tablet compositions that are generally dry until chewed. During chewing, the pressed gum tablet consolidates with saliva into a soft chewy substance, thereby releasing the acid and base and producing effervescence. Moreover, pressed gum tablets are generally more porous than molten gum base, thereby providing better release of the effervescent system components.

### SUMMARY

There is provided a chewing gum composition comprising:
a particulate chewing gum base;
a tableting powder; and
an effervescent system,
wherein said chewing gum base and said tableting powder comprise a tableted gum layer and
said effervescent system comprises at least one effervescent layer in contact with said tableted gum layer.

As used herein the transitional term "comprising," (also "comprises," etc.) which is synonymous with "including, " "containing," or "characterized by," is inclusive or open-ended and does not exclude additional, unrecited elements or method steps, regardless of its use in the preamble or the body of the claim.

As used herein, the terms "bubble gum" and "chewing gum" are used interchangeably and are both meant to include any gum compositions.

Embodiments described herein provide a chewing gum composition that effervesces when chewed by a user. Desirably, the chewing gum composition is in the form of a pressed gum tablet. The chewing gum composition may include a particulate chewing gum base, a tableting powder and an effervescent system.

### Particulate Gum Base and Tableting Powder

The gum base used in the chewing gum compositions of the present invention may be any conventional chewing gum base used in making chewing gum. As opposed to molten, or thermoplastic, gum base, however, the gum base in the compositions of the present invention is in a particulate form, such as, but not limited to, a powdered or granular gum base. The particulate gum base may be essentially free of water and can readily be formed into any desired shape, such as by compression.

The gum base may include any component known in the chewing gum art. For example, the gum base may include elastomers, bulking agents, waxes, elastomer solvents, emulsifiers, plasticizers, fillers and mixtures thereof.

The elastomers (rubbers) employed in the gum base will vary greatly depending upon various factors such as the type of gum base desired, the consistency of gum composition desired and the other components used in the composition to make the final chewing gum product. The elastomer may be any water-insoluble polymer known in the art, and includes those gum polymers utilized for chewing gum and bubble gums. Illustrative examples of suitable polymers in gum bases include both natural and synthetic elastomers. For example, those polymers which are suitable in gum base compositions include, without limitation, natural substances (of vegetable origin) such as chicle, natural rubber, crown gum, nispero, rosindinha, jelutong, perillo, niger gutta, tunu, balata, guttapercha, lechi capsi, sorva, gutta kay, and the like, and mixtures thereof. Examples of synthetic elastomers include, without limitation, styrene-butadiene copolymers (SBR), polyisobutylene, isobutylene-isoprene copolymers, polyethylene, polyvinyl acetate and the like, and mixtures thereof.

The amount of elastomer employed in the gum base may vary depending upon various factors such as the type of gum base used, the consistency of the gum composition desired and the other components used in the composition to make the final chewing gum product. In general, the elastomer will be present in the gum base in an amount from about 10% to about 60% by weight, desirably from about 35% to about 40% by weight.

In some embodiments, the gum base may include wax. It softens the polymeric elastomer mixture and improves the elasticity of the gum base. When present, the waxes employed will have a melting point below about 60°C, and preferably between about 45°C and about 55°C. The low melting wax may be a paraffin wax. The wax may be present in the gum base in an amount from about 6% to about 10%, and preferably from about 7% to about 9.5%, by weight of the gum base.

In addition to the low melting point waxes, waxes having a higher melting point may be used in the gum base in amounts up to about 5%, by weight of the gum base. Such high melting waxes include beeswax, vegetable wax, candelilla wax, carnuba wax, most petroleum waxes, and the like, and mixtures thereof.

In addition to the components set out above, the gum base may include a variety of other ingredients, such as components selected from elastomer solvents, emulsifiers, plasticizers, fillers, and mixtures thereof.

The gum base may contain elastomer solvents to aid in softening the elastomer component. Such elastomer solvents may include those elastomer solvents known in the art, for example, terpinene resins such as polymers of alpha-pinene or beta-pinene, methyl, glycerol and pentaerythritol esters of rosins and modified rosins and gums such as hydrogenated, dimerized and polymerized rosins, and mixtures thereof. Examples of elastomer solvents suitable for use herein may include the pentaerythritol ester of partially hydrogenated wood and gum rosin, the pentaerythritol ester of wood and gum rosin, the glycerol ester of wood rosin, the glycerol ester of partially dimerized wood and gum rosin, the glycerol ester of polymerized wood and gum rosin, the glycerol ester of tall oil rosin, the glycerol ester of wood and gum rosin and the partially hydrogenated wood and gum rosin and the partially hydrogenated methyl ester of wood and rosin, and the like, and mixtures thereof. The elastomer solvent may be employed in the gum base in amounts from about 2% to about 15%, and preferably from about 7% to about 11%, by weight of the gum base.

The gum base may also include emulsifiers which aid in dispersing the immiscible components into a single stable system. The emulsifiers useful in this invention include glyceryl monostearate, lecithin, fatty acid monoglycerides, diglycerides, propylene glycol monostearate, and the like, and mixtures thereof. The emulsifier may be employed in amounts from about 2% to about 15%, and more specifically, from about 7% to about 11%, by weight of the gum base.

The gum base may also include plasticizers or softeners to provide a variety of desirable textures and consistency properties. Because of the low molecular weight of these ingredients, the plasticizers and softeners are able to penetrate the fundamental structure of the gum base making it plastic and less viscous. Useful plasticizers and softeners include lanolin, palmitic acid, oleic acid, stearic acid, sodium stearate, potassium stearate, glyceryl triacetate, glyceryl lecithin, glyceryl monostearate, propylene glycol monostearate, acetylated monoglyceride, glycerine, and the like, and mixtures thereof. Waxes, for example, natural and synthetic waxes, hydrogenated vegetable oils, petroleum waxes such as polyurethane waxes, polyethylene waxes, paraffin waxes, microcrystalline waxes, fatty waxes, sorbitan monostearate, tallow, propylene glycol, mixtures thereof, and the like, may also be incorporated into the gum base. The plasticizers and softeners are generally employed in the gum base in amounts up to about 20% by weight of the gum base, and more specifically in amounts from about 9% to about 17%, by weight of the gum base.

Plasticizers also include hydrogenated vegetable oils, such as soybean oil and cottonseed oils, which may be employed alone or in combination. These plasticizers provide the gum base with good texture and soft chew characteristics. These plasticizers and softeners are generally employed in amounts from about 5% to about 14%, and more specifically in amounts from about 5% to about 13.5%, by weight of the gum base.

Anhydrous glycerin also be employed as a softening agent, such as the commercially available United States Pharmacopeia (USP) grade. Glycerin is a syrupy liquid with a sweet warm taste and has a sweetness of about 60% of that of cane sugar. Because glycerin is hygroscopic, the anhydrous glycerin may be maintained under anhydrous conditions throughout the preparation of the chewing gum composition.

In some embodiments, the gum base of this invention may also include effective amounts of bulking agents such as mineral adjuvants which may serve as fillers and textural agents. Useful mineral adjuvants include calcium carbonate, magnesium carbonate, alumina, aluminum hydroxide, aluminum silicate, talc, tricalcium phosphate, dicalcium phosphate, calcium sulfate and the like, and mixtures thereof. These fillers or adjuvants may be used in the gum base compositions in various amounts. Preferably the amount of filler, when used, will be present in an amount from about 15% to about 40%, and desirably from about 20% to about 30%, by weight of the gum base.

A variety of traditional ingredients may be optionally included in the gum base in effective amounts such as flavor agents and coloring agents described above, antioxidants, preservatives, and the like. For example, titanium dioxide and other dyes suitable for food, drug and cosmetic applications, known as F. D. & C. dyes, may be utilized. An anti-oxidant such as butylated hydroxytoluene (BHT), butylated hydroxyanisole (BHA), propyl gallate, vitamin E and mixtures thereof, may also be included. Other conventional chewing gum additives known to one having ordinary skill in the chewing gum art may also be used in the gum base.

The chewing gum compositions may include amounts of conventional additives selected from the group consisting of sweetening agents, plasticizers, softeners, emulsifiers, waxes, fillers, bulking agents (carriers, extenders, bulk sweeteners), mineral adjuvants, flavor agents and coloring agents discussed above, antioxidants, acidulants, thickeners, medicaments, and the like, and mixtures thereof. Some of these additives may serve more than one purpose. For example, in sugarless gum compositions, a sweetener, such as maltitol or other sugar alcohol, may also function as a bulking agent.

Bulk sweeteners, such as sugars, sugarless bulk sweeteners, or the like, or mixtures thereof, generally are present in amounts of about 5% to about 95% by weight of the chewing gum composition.

Suitable sugar sweeteners generally include mono-saccharides, di-saccharides and poly-saccharides such as but not limited to, sucrose (sugar), dextrose, maltose, dextrin, xylose, ribose, glucose, mannose, galactose, fructose (levulose), invert sugar, fructo oligo saccharide syrups, partially hydrolyzed starch, corn syrup solids and mixtures thereof.

Suitable sugarless bulk sweeteners include sugar alcohols (or polyols) such as, but not limited to, sorbitol, xylitol, mannitol, galactitol, maltitol, hydrogenated isomaltulose (ISOMALT), lactitol, erythrytol, hydrogenated starch hydrolysate, stevia and mixtures thereof.

Suitable hydrogenated starch hydrolysates include those disclosed in U.S. Pat. No. 4,279,931 and various hydrogenated glucose syrups and/or powders which contain sorbitol, hydrogenated disaccharides, hydrogenated higher polysaccharides, or mixtures thereof. Hydrogenated starch hydrolysates are primarily prepared by the controlled catalytic hydrogenation of corn syrups. The resulting hydrogenated starch hydrolysates are mixtures of monomeric, dimeric, and polymeric saccharides. The ratios of these different saccharides give different hydrogenated starch hydrolysates different properties. Mixtures of hydrogenated starch hydrolysates, such as LYCASIN, a commercially available product manufactured by Roquette Freres of France, and HYSTAR, a commercially available product manufactured by Lonza, Inc., of Fairlawn, N.J., are also useful.

In some embodiments, high-intensity sweeteners also may be included in the confectionery compositions. Without being limited to particular sweeteners, representative categories and examples include:
(a) water-soluble artificial sweeteners such as soluble saccharin salts, i.e., sodium or calcium saccharin salts, cyclamate salts, the sodium, ammonium or calcium salt of 3,4-dihydro-6-methyl-1,2,3-oxathiazine-4-one-2,2-dioxide, the potassium salt of 3,4-dihydro-6-methyl-1,2,3-oxathiazine-4-one-2,2-dioxide (Acesulfame-K), the free acid form of saccharin, and mixtures thereof;
(b) dipeptide based sweeteners, such as L-aspartic acid derived sweeteners, such as L-aspartyl-L-phenylalanine methyl ester (Aspartame) and materials described in U.S. Pat. No. 3,492,131, L-alphaaspartyl-N-(2,2,4,4-tetramethyl-3-thietanyl)-D-alaninamide hydrate (Alitame), methyl esters of L-aspartyl-L-phenylglycerine and L-aspartyl-L-2,5-dihydrophenyl-glycine, L-aspartyl-2,5-dihydro-L-phenylalanine; L-aspartyl-L-(1-cyclohexen)-alanine, and mixtures thereof;
(c) water-soluble sweeteners derived from naturally occurring water-soluble sweeteners, such as chlorinated derivatives of ordinary sugar (sucrose), e.g., chlorodeoxysugar derivatives such as derivatives of chlorodeoxysucrose or chlorodeoxygalactosucrose, known, for example, under the product designation of Sucralose; examples of chlorodeoxysucrose and chlorodeoxygalactosucrose derivatives include but are not limited to: 1-chloro-1'-deoxysucrose; 4-chloro-4-deoxy-alpha-D-galactopyranosyl-alpha-D-fructofuranoside, or 4-chloro-4-deoxygalactosucrose; 4-chloro-4-deoxy-alpha-D-galactopyranosyl-1-chloro-1-deoxy-beta-D-fructo-furanoside, or 4,1'-dichloro-4,1'-dideoxygalactosucrose; 1',6'-dichloro1',6'-dideoxysucrose; 4-chloro-4-deoxy-alpha-D-galactopyranosyl-1,6-dichloro-1,6-dideoxy-beta-D- fructofuranoside, or 4,1',6'-trichloro-4,1',6'-trideoxygalactosucrose; 4,6-dichloro-4,6-dideoxy-alpha-D-galactopyranosyl-6-chloro-6-deoxy-beta-D- fructofuranoside, or 4,6,6'-trichloro-4,6,6'-trideoxygalactosucrose; 6,1',6'-trichloro-6,1',6'-trideoxysucrose; 4,6-dichloro-4,6-dideoxy-alpha-D-galacto-pyranosyl-1,6-dichloro-1,6-dideox y-beta-D-fructofuranoside, or 4,6,1',6'-tetrachloro4,6,1',6'-tetradeoxygalacto-sucrose; and 4,6,1',6'-tetradeoxy-sucrose, and mixtures thereof; and
(d) protein based sweeteners such as thaumaoccous danielli (Thaumatin I and II).

The intense sweetening agents may be used in many distinct physical forms well-known in the art to provide an initial burst of sweetness and/or a prolonged sensation of sweetness. Without being limited thereto, such physical forms include free forms, such as spray dried, powdered, beaded forms, encapsulated forms, and mixtures thereof.

In general, an effective amount of intense sweetener may be utilized to provide the level of sweetness desired, and this amount may vary with the sweetener selected. The intense sweetener may be present in amounts from about 0.001% to about 3%, by weight of the composition, depending upon the sweetener or combination of sweeteners used. The exact range of amounts for each type of sweetener may be selected by those skilled in the art.

The plasticizers, softening agents, mineral adjuvants, waxes and antioxidants discussed above, as being suitable for use in the gum base, may also be used in the chewing gum composition. Examples of other conventional additives which may be used include emulsifiers, such as lecithin and glyceryl monostearate, thickeners, used alone or in combination with other softeners, such as methyl cellulose, alginates, carrageenan, xanthan gum, gelatin, carob, tragacanth, locust bean, and carboxy methyl cellulose, acidulants such as malic acid, adipic acid, citric acid, tartaric acid, fumaric acid, and mixtures thereof, and fillers, such as those discussed above under the category of mineral adjuvants.

Other conventional gum additives known to one having ordinary skill in the chewing gum art also may be used in the chewing gum compositions.

The particulate gum base may be formed using standard grinding techniques known in the art. The starting material may be any conventional gum base, such as those used to produce molten gum bases. The particulate gum base may be formed, for example, by shredding, grinding or crushing the gum base or other processes, as described in U.S. Patent Nos. 3,262,784, 4,405,647, 4,753,805 and 6,290,985 and U.S. Publication No. 2003/00276871.

Desirably, the particulate gum base is ground or the like into a particulate form that is similar in size to the tableting powder. By using components of like particle size, a homogenous mix of gum base and tableting powder may be achieved, which may provide a gum tablet of similar homogenous make-up. The gum base and tableting powder may have a particle size of about 4 to about 100 mesh, desirably about 8 to about 25 mesh, and more desirably about 12 to about 20 mesh.

The particulate gum base may be present in amounts of about 10% to about 80% by weight of the chewing gum composition, or tablet, desirably about 20% to about 50% by weight, and more desirably about 30% to about 40% by weight.

The particulate gum base may be combined with a tableting powder to form the pressed gum tablet. The tableting powder desirably is in a dry, finely-divided form. Desirable particle size is provided above. The tableting powder may be a sucrose-based, dextrose-based or polyol-based powder, or combinations thereof. For example, the polyol-based powder may be a sorbitol or mannitol powder. The tableting powder may include other optional ingredients, such as flavor agents, color agents, sugar and/or sugarless sweeteners, and the like and combinations thereof.

In some embodiments, it may be desirable to combine a food-grade lubricant with the particulate gum base and tableting powder. Food-grade lubricants may assist in processing the gum composition into pressed tablets. More specifically, lubricants are used to prevent excess wear on dies and punches in tableting manufacture. Lubricants may be useful immediately after compression of the tablet within the die to reduce friction between the tablet and inner die wall.

The food-grade lubricant may be added separately or it may be included with the tableting powder, as in some commercially available tableting powders. Examples of suitable food-grade lubricants include: metallic stearates; fatty acids; hydrogenated vegetable oil; partially hydrogenated vegetable oils; animal fats; polyethylene glycols; polyoxyethylene monostearate; talc; silicon dioxide; and combinations thereof. Food-grade lubricants may be present in amounts of about 0-6% by weight of the gum composition.

### Effervescent System

The chewing gum compositions of the present invention also include an effervescent system. The effervescent system includes an edible acid and a base, which react upon chewing to generate effervescence.

The base may be selected from, but is not limited to, alkali metal carbonates, alkali metal bicarbonates, alkaline earth metal carbonates, alkaline earth metal bicarbonates and combinations thereof. The edible acid may be selected from, but is not limited to, citric acid, phosphoric acid, tartaric acid, malic acid, ascorbic acid or combinations thereof.

In some embodiments, the edible acid and/or base may be encapsulated. Encapsulation may be desirable because it lends further moisture-resistance to the components, thereby preventing premature reaction of the acid and base components. The acid and base may be encapsulated as microcapsules or microparticles as described in PCT Publication No. WO 2004/064544. Suitable encapsulants include, but are not limited to, fats, polymers, carbohydrates and combinations thereof. A particularly suitable encapsulant is gum arabic.

The effervescent system may further include an active component to be delivered to the oral cavity. In particular, carbon dioxide released during effervescence of the chewing gum dilates the oral membranes of the user. This dilation may assist active components having low solubility, i.e., hydrophobic, in penetrating the oral membranes. As such, carbon dioxide released during effervescence may increase the bioavailability of an active component delivered with the effervescent system.

Active components desirable for delivery with the effervescent system include, but are not limited to, flavor agents and oral care actives. Other active components known to those skilled in the art, such as hydrophobic drugs, are considered well within the scope of the present invention.

Flavor agents which may be used include those flavors known to the skilled artisan, such as natural and artificial flavors. These flavorings may be chosen from synthetic flavor oils and flavoring aromatics and/or oils, oleoresins and extracts derived from plants, leaves, flowers, fruits, and so forth, and combinations thereof. Nonlimiting representative flavor oils include spearmint oil, cinnamon oil, oil of wintergreen (methyl salicylate), peppermint oil, clove oil, bay oil, anise oil, eucalyptus oil, thyme oil, cedar leaf oil, oil of nutmeg, allspice, oil of sage, mace, oil of bitter almonds, and cassia oil. Also useful flavorings are artificial, natural and synthetic fruit flavors such as vanilla, and citrus oils including lemon, orange, lime, grapefruit, and fruit essences including apple, pear, peach, grape, strawberry, raspberry, cherry, plum, pineapple, apricot and so forth. These flavoring agents may be used in liquid or solid form and may be used individually or in admixture. Commonly used flavors include mints such as peppermint, menthol, spearmint, artificial vanilla, cinnamon derivatives, and various fruit flavors, whether employed individually or in admixture.

Other useful flavorings include aldehydes and esters such as cinnamyl acetate, cinnamaldehyde, citral diethylacetal, dihydrocarvyl acetate, eugenyl formate, p-methylamisol, and so forth may be used. Generally any flavoring or food additive such as those described in Chemicals Used in Food Processing, publication 1274, pages 63-258, by the National Academy of Sciences, may be used.

Further examples of aldehyde flavorings include but are not limited to acetaldehyde (apple), benzaldehyde (cherry, almond), anisic aldehyde (licorice, anise), cinnamic aldehyde (cinnamon), citral, i.e., alpha-citral (lemon, lime), neral, i.e., beta-citral (lemon, lime), decanal (orange, lemon), ethyl vanillin (vanilla, cream), heliotrope, i.e., piperonal (vanilla, cream), vanillin (vanilla, cream), alpha-amyl cinnamaldehyde (spicy fruity flavors), butyraldehyde (butter, cheese), valeraldehyde (butter, cheese), citronellal (modifies, many types), decanal (citrus fruits), aldehyde C-8 (citrus fruits), aldehyde C-9 (citrus fruits), aldehyde C-12 (citrus fruits), 2-ethyl butyraldehyde (berry fruits), hexenal, i.e., trans-2 (berry fruits), tolyl aldehyde (cherry, almond), veratraldehyde (vanilla), 2,6-dimethyl-5-heptenal, i.e., melonal (melon), 2,6-dimethyloctanal (green fruit), and 2-dodecenal (citrus, mandarin), cherry, grape, strawberry shortcake, and mixtures thereof.

In some embodiments, the flavor agent may be employed in either liquid form and/or dried form. When employed in the latter form, suitable drying means such as spray drying the oil may be used. Alternatively, the flavor agent may be absorbed onto water soluble materials, such as cellulose, starch, sugar, maltodextrin, gum arabic and so forth or may be encapsulated. The actual techniques for preparing such dried forms are well-known.

In some embodiments, the flavor agents may be used in many distinct physical forms well-known in the art to provide an initial burst of flavor and/or a prolonged sensation of flavor. Without being limited thereto, such physical forms include free forms, such as spray dried, powdered, beaded forms, encapsulated forms, and mixtures thereof.

Oral care actives which may be used include those actives known to the skilled artisan, such as, but not limited to, surfactants, breath freshening agents, anti-microbial agents, antibacterial agents, anti-calculus agents, anti-plaque agents, oral malodor control agents, fluoride compounds, quaternary ammonium compounds and combinations thereof.

Suitable surfactants include, but are not limited to, salts of fatty acids selected from the group consisting of C₈-C₂₄, palmitoleic acid, oleic acid, eleosteric acid, butyric acid, caproic acid, caprylic acid, capric acid, lauric acid, myristic acid, palmitic acid, stearic acid, ricinoleic acid, arachidic acid, behenic acid, lignoceric acid, cerotic acid, sulfated butyl oleate, medium and long chain fatty acid esters, sodium oleate, salts of fumaric acid, potassium glomate, organic acid esters of mono- and diglycerides, stearyl monoglyceridyl citrate, succistearin, dioctyl sodium sulfosuccinate, glycerol tristearate, lecithin, hydroxylated lecithin, sodium lauryl sulfate, acetylated monoglycerides, succinylated monoglycerides, monoglyceride citrate, ethoxylated mono- and diglycerides, sorbitan monostearate, calcium stearyl-2-lactylate, sodium stearyl lactylate, lactylated fatty acid esters of glycerol and propylene glycerol, glycerol-lactoesters of C₈-C₂₄ fatty acids, polyglycerol esters of C₈-C₂₄ fatty acids, propylene glycol alginate, sucrose C₈-C₂₄ fatty acid esters, diacetyl tartaric and citric acid esters of mono- and diglycerides, triacetin, sarcosinate surfactants, isethionate surfactants, tautate surfactants, pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and combinations thereof.

Suitable antibacterial agents include, but are not limited to, chlorhexidine, alexidine, quaternary ammonium salts, benzethonium chloride, cetyl pyridinium chloride, 2,4,4'-trichloro-2'-hydroxy-diphenyl ether (triclosan) and combinations thereof.

Suitable fluoride compounds include, but are not limited to, sodium fluoride, sodium monofluorophosphate, stannous fluoride and combinations thereof.

Suitable anti-calculus agents include, but are not limited to, pyrophosphates, triphosphates, polyphosphates, polyphosphonates, dialkali metal pyrophosphate salt, tetra alkali polyphosphate salt, tetrasodium pyrophosphate, tetrapotassium pyrophosphate, sodium tripolyphosphate and combinations thereof.

Suitable anti-microbial agents include, but are not limited to, cetylpyridinium chloride, zinc compounds, copper compounds and combinations thereof.

Other oral care activies known to those skilled in the art are considered well within the scope of the present invention.

The effervescent system may be present in amounts of about 0.5% to about 80% by weight of the chewing gum composition, or tablet, desirably about 1% to about 20"% by weight, and more desirably about 5% to about 20% by weight.

Some embodiments of the present invention provide an effervescent system premix for use in manufacturing pressed chewing gum tablets. The premix may include a base and edible acid, as described above, as well as an active agent for delivery to the oral cavity.

### Delivery System

As described above, the chewing gum composition desirably is in the form of a pressed gum tablet, where the particulate chewing gum base, tableting powder and effervescent system are pressed into a tablet form. Upon chewing, the pressed gum tablet consolidates into a soft chewy substance. This pressed gum tablet does not, however, form part of the present invention.

The chewing gum composition may be a single-layer pressed tablet having the gum base, tableting powder and effervescent system all in one layer; however, this does not form part of the present invention.

The chewing gum composition is a multi-layer pressed tablet. In some multi-layer tablets, the effervescent system may be in a separate layer from the gum base and tableting powder layer, i.e., the gum layer. As such, these have at least one separate gum layer and effervescent layer. The effervescent layer may be in contact with the gum layer.

In other multi-layer tablets, an effervescent system also may be present in the layer containing the gum base and tableting powder. As such, this provides a multi-layer pressed gum tablet having effervescence in the gum layer as well as a separate effervescent layer.

Multi-layer tablets may have any desirable number of layers. For example, a tablet may have a single gum layer in contact with a single effervescent layer. Alternatively, a gum layer may be positioned between two effervescent layers or vice-versa.

The pressed gum tablet also may have a coating layer surrounding the tablet. The coating layer may contain any ingredients conventionally used in the chewing gum art. For instance, the coating may contain sugar, sugarless or high intensity sweeteners or the like, coloring agents, flavor agents and warming and/or cooling agents, among others. The coating layer also may include an effervescent system, as described above.

The chewing gum compositions desirably have a very low moisture content. Accordingly, some embodiments have a total water content of greater than about 0% to about 5% by w eight of the composition. The density of the composition, may be about 0.2 to about 1.3 g/cc. Further, the compositions, or tablets, may have a dissolution rate of about 1 to about 20 minutes. When in a pressed tablet form, the chewing gum may have a Shore hardness of about 30 to about 200.

In single-layer tablets, the powder batch may be pressed into gum tablets as described above. However, this does not form part of the present invention.

In multi-layer tablets, a separate effervescent layer may be formed by combining effervescent system components and any other desirable additives and blending to the desired powder consistency. The gum layer batch, which as described above contains effervescent system components, and the separate effervescent layer batch may be filled into the tableting machine in sequence and pressed together to form a multi-layer gum tablet.

Alternatively, the gum layer batch may be formed without the effervescent system components. A separate effervescent layer batch may be prepared, as described above. The gum layer and effervescent layer batches may be filled into the tableting machine in sequence and pressed together to form a multi-layer tablet.

Any number of powder batches may be filled into the tableting machine in any sequence and compressed together to form tablets having any desired number of layers.

In other multi-layer tablets, a first layer, which may be either a gum layer or an effervescent layer or a combination thereof, is filled in the tableting machine and pressed into a tablet layer. The powder batch for the second layer may be filled on top of the first pressed layer. A second compression forms the multi-layer tablet. Additional compressions may be performed to add additional layers.

The features and advantages of the present invention are more fully shown by the following examples which are provided for purposes of illustration, and are not to be construed as limiting the invention in any way.

### EXAMPLES

### Example 1: (not part of the invention)

**Table 1: Single-Layer Effervescent Pressed Gum Tablet**

| Component | % by weight |
|---|---|
| Powdered gum base/sorbitol | 60.4 |
| Sorbitol | 14 |
| Sodium bicarbonate (granular) | 12 |
| Flavor | 2 |
| Sucralose | 0.3 |
| Citric acid (granular | 7 |
| Silicon dioxide | 0.3 |
| Magnesium stearate | 4 |

An effervescent single-layer chewing gum tablet was prepared according to the formulation in Table 1 above.

The powdered gum base and sorbitol were combined with the sodium bicarbonate, citric acid, sucralose and flavor. The combination was blended for about twelve minutes. The batch then was passed through a size 14 mesh screen. Silicon dioxide was added to the screened batch and the batch was blended for about five minutes. The magnesium stearate was added to the batch in two portions (2% each). After each portion of magnesium stearate was added, the batch was blended for about five minutes until the desirable powdered consistency was achieved. The batch then was filled into the compression apparatus (Piccola Model D-8 mini rotary tablet press) and compressed into a gum tablet.

**Table 2: Multi-Layer Effervescent Pressed Gum Tablet**

| Component | % by weight |
|---|---|
| **Effervescent Gum Layer** | |
| Powdered gum base/sorbitol | 42.28 |
| Sorbitol | 9.80 |
| Sodium bicarbonate (granular) | 8.40 |
| Flavor | 1.40 |
| Sucralose | 0.21 |
| Citric acid (granular) | 4.90 |
| Silicon dioxide | 0.21 |
| Magnesium stearate | 2.80 |
| | |

| **Effervescent Tablet Layer** | |
|---|---|
| Citric acid (granular) | 11.70 |
| Sodium bicarbonate (granular) | 16.50 |
| Flavor | 1.50 |
| Magnesium stearate | 0.30 |

An effervescent multi-layer chewing gum tablet was prepared according to the formulation in Table 2 above.

The effervescent gum layer components were combined and blended as described in Example 1. The effervescent tablet layer components similarly were combined and blended. The powdered batches were filled in the compression apparatus (Piccola Model D-8 mini rotary tablet press) in sequence and compressed together to form a bi-layer tablet.

**Table 3: Multi-Layer Effervescent Pressed Gum Tablet**

| Component | % by weight |
|---|---|
| **Non-Effervescent Gum Laver** | |
| Powdered gum base/sorbitol | 55.58 |
| Sorbitol | 9.80 |
| Flavor | 1.40 |
| Sucralose | 0.21 |
| Silicon dioxide | 0.21 |
| Magnesium stearate | 2.80 |
| | |

| **Effervescent Tablet Layer** | |
|---|---|
| Citric acid (granular) | 11.70 |
| Sodium bicarbonate (granular) | 16.50 |
| Flavor | 1.50 |
| Magnesium stearate | 0.30 |

An effervescent multi-layer chewing gum tablet was prepared according to the formulation in Table 3 above.

The gum layer components were combined and blended as described in Example 1, however, without any effervescent system components in the batch, as indicated in Table 3 above. The effervescent tablet layer components similarly were combined and blended. The powdered batches were filled in the compression apparatus (Piccola Model D-8 mini rotary tablet press) in sequence and compressed together to form a bi-layer tablet.

## Claims

1. A chewing gum composition comprising:
a particulate chewing gum base;
a tableting powder; and
an effervescent system,
wherein said chewing gum base and said tableting powder comprise a tableted gum layer and
said effervescent system comprises at least one effervescent layer in contact with said tableted gum layer.

2. The chewing gum composition according to claim 1, wherein said particulate chewing gum base is essentially free of water.

3. The chewing gum composition according to claim 1, wherein said particulate chewing gum base comprises a powder.

4. The chewing gum composition according to claim 1, wherein said tableting powder is selected from sucrose powder; dextrose powder; polyol powder; and combinations thereof.

5. The chewing gum composition according to claim 4, wherein said polyol comprises sorbitol.

6. The chewing gum composition according to claim 1, wherein said effervescent system comprises:
a base selected from alkali metal carbonates; alkali metal bicarbones; alkaline earth metal carbonates; alkaline earth metal bicarbonates; and combinations thereof; and
an edible acid.

7. The chewing gum composition according to claim 6, wherein said edible acid is selected from citric acid; phosphoric acid; tartaric acid; malic acid; ascorbic acid; and combinations thereof.

8. The chewing gum composition according to claim 6, wherein said edible acid is encapsulated.

9. The chewing gum composition according to claim 8, wherein the encapsulant is selected from a fat, a polymer, a carbohydrate and combinations thereof.

10. The chewing gum composition according to claim 6, wherein said base is encapsulated.

11. The chewing gum composition according to claim 10, wherein the encapsulant is selected from the group consisting from a fat, a polymer, a carbohydrate and combinations thereof.

12. The chewing gum composition according to claim 6, wherein said effervescent system further comprises at least one flavor agent.

13. The chewing gum composition according to claim 12, wherein said flavor agent is selected from flavor oils; flavoring aromatics and/or oils; oleoresins; extracts derived from plants, leaves, flowers or fruits; derivatives and combinations thereof.

14. The chewing gum composition according to claim 12, wherein said flavor agent is selected from spearmint oil; cinnamon oil; oil of wintergreen; peppermint oil; clove oil; bay oil; anise oil; eucalyptus oil; thyme oil; cedar leaf oil; oil of nutmeg; allspice; oil of sage; mace; oil of bitter almonds; cassia oil; vanilla; citrus oils including lemon, orange, lime and grapefruit; fruit essences including apple, pear, peach, grape, strawberry, raspberry, cherry, plum, pineapple, and apricot; menthol; cinnamon derivatives; cinnamyl acetate; cinnamaldehyde; citral diethylacetal; dihydrocarvyl acetate; eugenyl formate; p-methylamisol; acetaldehyde; benzaldehyde; anisic aldehyde; cinnamic aldehyde; alpha-citral; beta-citral; decanal; ethyl vanillin; piperonal; vanillin; alpha-amyl cinnamaldehyde; butyraldehyde; valeraldehyde; citronellal; aldehyde C-8; aldehyde C-9; aldehyde C-12; 2-ethyl butyraldehyde; trans-2; tolyl aldehyde; veratraldehyde; 2,6-dimethyl-5-heptenal; 2,6-dimethyloctanal; 2-dodecenal; cherry; grape; strawberry shortcake; and mixtures thereof.

15. The chewing gum composition according to claim 6, wherein said effervescent system further comprises at least one oral care active.

16. The chewing gum composition according to claim 15, wherein said oral care active is selected from surfactants; breath freshening agents; anti-microbial agents; antibacterial agents; anti-calculus agents; anti-plaque agents; oral malodor control agents; fluoride compounds; quaternary ammonium compounds; and combinations thereof.

17. The chewing gum composition according to claim 16, wherein said surfactant is selected from salts of fatty acids selected from C₈-C₂₄, palmitoleic acid, oleic acid, eleosteric acid, butyric acid, caproic acid, caprylic acid, capric acid, lauric acid, myristic acid, palmitic acid, stearic acid, ricinoleic acid, arachidic acid, behenic acid, lignoceric acid, cerotic acid, sulfated butyl oleate, medium and long chain fatty acid esters, sodium oleate, salts of fumaric acid, potassium glomate, organic acid esters of mono- and diglycerides, stearyl monoglyceridyl citrate, succistearin, dioctyl sodium sulfosuccinate, glycerol tristearate, lecithin, hydroxylated lecithin, sodium lauryl sulfate, acetylated monoglycerides, succinylated monoglycerides, monoglyceride citrate, ethoxylated mono- and diglycerides, sorbitan monostearate, calcium stearyl-2-lactylate, sodium stearyl lactylate, lactylated fatty acid esters of glycerol and propylene glycerol, glycerol-lactoesters of C₈-C₂₄ fatty acids, polyglycerol esters of C₈-C₂₄ fatty acids, propylene glycol alginate, sucrose C₈-C₂₄ fatty acid esters, diacetyl tartaric and citric acid esters of mono- and diglycerides, triacetin, sarcosinate surfactants, isethionate surfactants, tautate surfactants, pluronics, polyethylene oxide condensates of alkyl phenols, products derived from the condensation of ethylene oxide with the reaction product of propylene oxide and ethylene diamine, ethylene oxide condensates of aliphatic alcohols, long chain tertiary amine oxides, long chain tertiary phosphine oxides, long chain dialkyl sulfoxides, and combinations thereof.

18. The chewing gum composition according to claim 16, wherein said antibacterial agent is selected from chlorhexidine; alexidine; quaternary ammonium salts; benzethonium chloride; cetyl pyridinium chloride; 2,4,4'-trichloro-2'-hydroxy-diphenyl ether (triclosan); and combinations thereof.

19. The chewing gum composition according to claim 16, wherein said fluoride compound is selected from the group consisting of: sodium fluoride; sodium monofluorophosphate; stannous fluoride; and combinations thereof.

20. The chewing gum composition according to claim 16, wherein said anti-calculus agent is selected from the group consisting of: pyrophosphates; triphosphates; polyphosphates; polyphosphonates; dialkali metal pyrophosphate salt; tetra alkali polyphosphate salt; tetrasodium pyrophosphate; tetrapotassium pyrophosphate; sodium tripolyphosphate; and combinations thereof.

21. The chewing gum composition according to claim 16, wherein said anti-microbial agent is selected from the group consisting of: cetylpyridinium chloride; zinc compounds; copper compounds; and combinations thereof.

22. The chewing gum composition according to claim 1, wherein said tableted gum layer further comprises a second effervescent system.

23. The chewing gum composition according to claim 1, wherein said tableted gum layer further comprises a flavor agent.

24. The chewing gum composition according to claim 1, further comprising one or more components selected from sweetening agents; flavor agents; coloring agents; physiological cooling agents; warming agents; and combinations thereof.

25. The chewing gum composition according to claim 1, wherein said particulate chewing gum base is present in amounts of 10% to 80% by weight of said chewing gum composition.

26. The chewing gum composition according to claim 1, wherein said effervescent system is present in amounts of 0.5% to 80% by weight of said chewing gum composition.

27. The chewing gum composition according to claim 1, further comprising a coating layer surrounding said chewing gum composition.

28. The chewing gum composition according to claim 1, wherein said composition has a total water content of greater than 0% to 5% by weight of said chewing gum composition.

29. The chewing gum composition according to claim 1, wherein said composition has a density of 0.2 to 1.3 g/cc.

30. The chewing gum composition according to claim 1, wherein said composition has a dissolution rate of 1 to 20 minutes.

## Patentansprüche

1. Kaugummizusammensetzung mit:
einem partikelförmigen Kaugummigrundstoff;
einem tablettierenden Pulver; und
einem schäumenden System;
wobei der Kaugummigrundstoff und das tablettierende Pulver eine tablettierte Gummischicht aufweise, und das schäumende System zumindest eine schäumende Schicht in Kontakt mit der tablettierten Gummischicht aufweist.

2. Kaugummizusammensetzung nach Anspruch 1,
wobei der partikelförmige Kaugummigrundstoff im Wesentlichen wasserfrei ist.

3. Kaugummizusammensetzung nach Anspruch 1,
wobei der partikelförmige Kaugummigrundstoff ein Pulver enthält.

4. Kaugummizusammensetzung nach Anspruch 1
wobei das tablettierende Pulver ausgewählt ist aus Sucrosepulver; Dextrosepulver; Polyolpulver; und Kombinationen davon.

5. Kaugummizusammensetzung nach Anspruch 4,
wobei das Polyol Sorbitol enthält.

6. Kaugummizusammensetzung nach Anspruch 1,
wobei das schäumende System aufweist:
einen Grundstoff ausgewählt aus Alkalimetallcarbonaten; Alkalimetallbicarbonaten; Erdalkalimetallcarbonaten; Erdalkalibicarbonaten ; und Kombinationen davon; und einer essbaren Säure.

7. Kaugummizusammensetzung nach Anspruch 6,
wobei die essbare Säure ausgewählt ist aus Zitronensäure; Phosphorsäure; Weinsäure; Äpfelsäure; Ascorbinsäure; und Kombinationen davon.

8. Kaugummizusammensetzung nach Anspruch 6,
wobei die essbare Säure verkapselt ist.

9. Kaugummizusammensetzung nach Anspruch 8,
wobei das Verkapselungsmittel ausgewählt ist aus einem Fett, einem Polymer, einem Kohlenwasserstoff und Kombinationen davon.

10. Kaugummizusammensetzung nach Anspruch 6,
wobei der Grundstoff verkapselt ist.

11. Kaugummizusammensetzung nach Anspruch 10,
wobei das Verkapselungsmittel ausgewählt ist aus der Gruppe bestehend aus einem Fett, einem Polymer, einem Kohlenwasserstoff und Kombinationen davon.

12. Kaugummizusammensetzung nach Anspruch 6,
wobei das schäumende System ferner zumindest einen Duftstoff enthält.

13. Kaugummizusammensetzung nach Anspruch 12,
wobei das Duftmittel ausgewählt ist aus Duftölen; duftenden Aromaten und/oder Ölen; Ölharzen; Extrakten erhalten aus Pflanzen, Blättern, Blumen oder Früchten; Derivaten und Kombinationen davon.

14. Kaugummizusammensetzung nach Anspruch 12,
wobei das Duftmittel ausgewählt ist aus Öl grüner Minze; Zimtöl; Öl von Wintergrün; Pfefferminzöl; Nelkenöl; Lorbeeröl; Anisöl; Eukalyptusöl; Thymianöl; Zedernblattöl; Muskatrtussöl; Piment; Salbeiöl; Morgenstern; Öl von Bittermandeln; Kassiaöl; Vanille; Zitrusölen einschließlich Zitrone, Orange, Limone und Grapefruit; Fruchtessenzen einschließlich Apfel, Birne, Pfirsich, Traube, Erdbeere, Himbeere, Kirsche, Pflaume, Ananas, und Aprikose; Menthol; Zimtderivaten; Zimtaldehyd; Citraldiethylacetal; Dihydrocarvylacetat; Eugenylformiat; p-Methyl-anisol; Acetaldehyd; Benzaldehyd; Anisaldehyd; Zimtaldehyd; Alpha-citral; Beta-citral; Dekanal; Ethylvanillin; Piperonal; Vanillin; Alpha-anylzimtaldehyd; Butylaldehyd; Valeraldehyd; Zitronellal; C8-Aldehyd; C9-Aldehyd; C12-Aldehyd; 2-Etylbutyraldehyd; Trans-2; Tolylaldehyd; Veratralaldehyd; 2,6-Dimethyl-5-heptenal; 2,6-Dimethyfoktanal; 2-Dodekenal; Kirsche; Traube; Erdbeerteekuchen; und Mischungen davon.

15. Kaugummizusammensetzung nach Anspruch 6,
wobei das schäumende System ferner zumindest einen Mundpflegewirkstoff enthält.

16. Kaugummizusammensetzung nach Anspruch 16,
wobei der Mundpflegewirkstoff ausgewählt ist aus Tensiden; Atemerfrischungsmittetn; antimikrobiellen Mitteln; antibakteriellen Mitteln; Antizahnsteinmitteln; Antiplaquemitteln; Mundgeruchbekämpfungsmitteln; Fluoridverbindungen; quartären Amaniumverbindungens und Kombinationen davon.

17. Kaugummizusammensetzung nach Anspruch 16,
wobei das Tensid ausgewählt ist aus Salzen von Fettsäuren ausgewählt von C₈-C₂₄, Palmitoleinsäure, Ölsäure, Eleosterinsäure, Buttersäure, Hexansäure, Octansäure, Decansäure, Laurinsäure, Myristinsäure, Palmitinsäure, Stearinsäure, Ricinolsäure, Arachidonsäure, Behensäure, Lignocerinsäure, Cerotinsäure, sulfatiertes Butyloleat, mittlere und langkettige Fettsäureester, Natriumoleat, Salze der Fumarsäure, Kaliumglomat, Ester organischer Säuren von Mono- und Diglyceriden, Stearylmonoglyceridylcitrat, Succistearin, Dioctylnatriumsulfosuccinat, Glycerintristearat, Lecithin, hydroxyliertes Lecithin, Natriumlaurylsulfat, acetylierte Monoglyceride, succinylierte Monoglyceride, Monoglyceridcitrat, ethaxylierte Mono- und Diglyceride, Sobitanmonostearat, Calciumstearyl-2-lactylat, Natriumstearyllactylat, lactylierte Fettsäureester von Glycerin und Propylenglycerin, Glycerinlactoester von C₈-C₂₄-Fettsäuren, Polyglycerinestern von C₈-C₂₄-Fettsäuren, Propylenglykolalginat, Sucrose-C₈-C₂₄-Fettsäureester, Diacetylweinsäure und Zitronensäureester von Mono- und Diglyceriden, Triacetin, Sarcosinattensiden, isothionattensiden, Tautattensiden, Poloxamere, Polyethylenoxidkondensate, von Alkylphenole, Produkten abgeleitet von der Kondensation von Ethylenoxid mit dem Reaktionsprodukt von Propylenoxid und Ethylendiamin, Ethylenoxidkondensaten von aliphatischen Alkoholen, langkettigen tertiären Aminoxiden, langkettigen tertiären Phosphinoxiden, langkettigen Dialkylsulfoxiden und Kombinationen davon.

18. Kaugummizusammensetzung nach Anspruch 16,
wobei das antibakterielle Mittel ausgewählt ist aus Chlorhexidin; Alexidin; quartären Ammoniumsalzen; Benzethoniumchlorid; Cetylpyridiniumchlorid; 2,4,4'-Trichloro-2'-hydroxy-diphenylether (Triclosan); und Kombinationen davon.

19. Kaugummizusammensetzung nach Anspruch 16,
wobei die Fluoridverbindung ausgewählt ist aus der Gruppe bestehend aus:
Natriumfluorid; Natriummonofluorphosphat; Zinnfluorid; und Kombinationen davon.

20. Kaugummizusammensetzung nach Anspruch 16,
wobei das Anti-Zahnsteinmittel ausgewählt ist aus der Gruppe bestehend aus:
Pyrophosphaten; Triphosphaten; Polyphosphaten; Polyphosphonaten; Dialkalimetallpyrophosphatsalz; Tetraalkalipolyphosphatsalz; Tetranatriumpyrophosphat; Tetrakaliumpyrophosphat; Natriumtripolyphosphat und Kombinationen davon.

21. Kaugummizusammensetzung nach Anspruch 16,
wobei das antimikrobielle Mittel ausgewählt ist aus der Gruppe bestehend aus:
Cetylpyridiniumchlorid; Zinkverbindungen; Kupferverbindungen; und Kombinationen davon.

22. Kaugummizusammensetzung nach Anspruch 1,
wobei die tablettierte Gummischicht ferner ein zweites schäumendes System enthält.

23. Kaugummizusammensetzung nach Anspruch 9,
wobei die tablettierte Gummischicht ferner einen Duftstoff enthält.

24. Kaugummizusammensetzung nach Anspruch 1,
ferner mit einem oder mehreren Bestandteilen ausgewählt aus Süßstoffen; Duftstoffen; Färbemitteln; physiologischen Kühlmitteln; Wärmemitteln und Kombinationen davon.

25. Kaugummizusammensetzung nach Anspruch 1,
wobei der teilchenförmige Kaugummigrundstoff in Mengen von 10 Gew.-% bis 80 Gew.-% der Kaugummizusammensetzung vorliegt.

26. Kaugummizusammensetzung nach Anspruch 1,
wobei das schäumende System in Mengen von 0,5 Gew.-% bis 80 Gew.-% der Kaugummizusammensetzung vorliegt.

27. Kaugummizusammensetzung nach Anspruch 1,
ferner mit einer Umhüllungsschicht, welche die Kaugummizusammensetzung umgibt.

28. Kaugummizusammensetzung nach Anspruch 1,
wobei die Zusammensetzung einen Gesamtwassergehalt von größer als 0 Gew.-% bis 5 Gew.-% der Kaugummizusammensetzung aufweist.

29. Kaugummizusammensetzung nach Anspruch 1,
wobei die Zusammensetzung eine Dichte von 0,2 bis 1,3 g/cc aufweist.

30. Kaugummizusammensetzung nach Anspruch 1,
wobei die Zusammensetzung eine Auflösungsgeschwindigkeit von 1 bis 20 Minuten hat.

## Revendications

1. Composition de gomme à mâcher comprenant:
une base de gomme à mâcher particulaire ;
une poudre pour fabrication de comprimés ; et
un système effervescent,
dans laquelle ladite base de gomme à mâcher et ladite poudre pour fabrication de comprimés comprennent une couche de gomme sous forme de comprimé et
ledit système effervescent comprend au moins une couche effervescente en contact avec ladite couche de gomme sous forme de comprimé.

2. Composition de gomme à mâcher selon la revendication 1, dans laquelle ladite base de gomme à mâcher particulaire est pratiquement exempte d'eau.

3. Composition de gomme à mâcher selon la revendication 1, dans laquelle ladite base de gomme à mâcher particulaire comprend une poudre.

4. Composition de gomme à mâcher selon la revendication 1, dans laquelle ladite poudre pour fabrication de comprimés est choisie parmi la poudre de saccharose, la poudre de dextrose, la poudre de polyol, et leurs combinaisons.

5. Composition de gomme à mâcher selon la revendication 4, dans laquelle ledit polyol comprend du sorbitol.

6. Composition de gomme à mâcher selon la revendication 1, dans laquelle ledit système effervescent comprend:
une base choisie parmi les carbonates de métal alcalin, les bicarbonates de métal alcalin, les carbonates de métal alcalino-terreux, les bicarbonates de métal alcalino-terreux, et leurs combinaisons, et
un acide comestible.

7. Composition de gomme à mâcher selon la revendication 6, dans laquelle ledit acide comestible est choisi parmi l'acide citrique, l'acide phosphorique, l'acide tartrique, l'acide malique, l'acide ascorbique, et leurs combinaisons.

8. Composition de gomme à mâcher selon la revendication 6, dans laquelle ledit acide comestible est encapsulé.

9. Composition de gomme à mâcher selon la revendication 8, dans laquelle le produit d'encapsulation est choisi parmi une graisse, un polymère, un hydrate de carbone et leurs combinaisons,

10. Composition de gomme à mâcher selon la revendication 6, dans laquelle ladite base est encapsulée.

11. Composition de gomme à mâcher selon la revendication 10, dans laquelle le produit d'encapsulation est choisi dans le groupe constitué par une graisse, un polymère, un hydrate de carbone et leurs combinaisons.

12. Composition de gomme à mâcher selon la revendication 6, dans laquelle ledit système effervescent comprend en outre au moins un agent de saveur.

13. Composition de gomme à mâcher selon la revendication 12, dans laquelle ledit agent de saveur est choisi parmi les huiles aromatiques ; les aromates et/ou huiles aromatiques ; les oléorésines ; les extraits dérivés de plantes, feuilles, fleurs ou fruits ; et leurs dérivés et combinaisons.

14. Composition de gomme à mâcher selon la revendication 12, dans laquelle ledit agent de saveur est choisi parmi l'huile de menthe verte ; l'huile de cannelle ; l'huile de wintergreen ; l'huile de menthe poivrée ; l'huile de clou de girofle ; l'huile de laurier-sauce ; l'huile d'anis ; l'huile d'eucalyptus ; l'huile de thym ; l'huile de feuilles de cèdre ; l'huile de noix de muscade ; le piment de la Jamaïque ; l'huile de sauge ; le macis ; l'huile d'amandes amères ; l'huile de cassie ; la vanille ; les huiles d'agrumes comprenant le citron, l'orange, le citron vert et le pamplemousse ; les essences de fruits comprenant la pomme, la poire, la pêche, le raisin, la fraise, la framboise, la cerise, la prune, l'ananas et l'abricot ; le menthol ; les dérivés de cannelle ; l'acétate de cinnamyle ; le cinnamaldéhyde ; le citral diéthylacétal ; l'acétate de dihydrocarvyle ; le formiate d'eugényle ; le p-méthylamisol ; l'acétaldéhyde ; le benzaldéhyde ; l'aldéhyde anisique ; l'aldéhyde cinnamique ; l'α-citral ; le β-citral ; le décanal ; l'éthylvanilline ; le pipéronal ; la vanilline ; l'α-amylcinnamaldéhyde ; le butyraldébyde ; le valéraldéhyde ; le citronellal ; l'aldéhyde C-8 ; l'aldéhyde C-9 ; l'aldéhyde C-12 ; le 2-éthylbutyraldéhyde ; le trans-2 ; le tolylaldéhyde ; le vératraldéhyde ; le 2,6-diméthyl-5-hepténal ; le 2,6-diméthyloctanal ; le 2-dodécénal ; la cerise ; le raisin ; le gâteau aux fraises ; et leurs mélanges.

15. Composition de gomme à mâcher selon la revendication 6, dans laquelle ledit système effervescent comprend en outre au moins un agent actif pour l'hygiène de la cavité orale.

16. Composition de gomme à mâcher selon la revendication 15, dans laquelle ledit agent actif pour l'hygiène de la cavité orale est choisi parmi les tensioactifs, les agents rafraichissant l'haleine, les agents antimicrobiens, les agents antibactériens, les agents antitartre, les agents anti-plaque, les agents luttant contre la mauvaise haleine, les composés fluorures, les composés d'ammonium quaternaire, et leurs combinaisons.

17. Composition de gomme à mâcher selon la revendication 16, dans laquelle ledit tensioactif est choisi parmi les sels d'acides gras choisis parmi ceux en C₈ à C₂₄, d'acide palmitoléique, d'acide oléique, d'acide éléostérique, d'acide butyrique, d'acide caproïque, d'acide caprylique, d'acide caprique, d'acide laurique, d'acide myristique, d'acide palmitique, d'acide stéarique, d'acide ricinoléique, d'acide arachidique, d'acide béhénique, d'acide lignocérique, d'acide cérotique, l'oléate de butyle sulfaté, les esters d'acides gras à chaîne moyenne et longue, l'oléate de sodium, les sels d'acide fumarique, le glomate de potassium, les esters d'acides organiques et de mono- et di-glycérides, le citrate de stéaryl-monoglycéridyle, la succistéarine, le dioctylsulfosuccinate de sodium, le tristéarate de glycérol, la lécithine, la lécithine hydroxylée, le laurylsulfate de sodium, les monoglycérides acétylés, les monoglycérides succinylés, le citrate de monoglycéride, les mono- et di-glycérides éthoxylés, le monostéarate de sorbitan, le stéaryl-2-lactylate de calcium, le stéaryl-lactylate de sodium, les esters lactylés d'acides gras de glycérol et de propylèneglycérol, les lactoesters de glycérol et d'acides gras en C₈ à C₂₄, les esters de polyglycérols et d'acides gras en C₈ à C₂₄, l'alginate de propylèneglycol, les esters d'acides gras en C₈ à C₂₄ et de saccharose, les esters d'acide diacétyltartrique et d'acide citrique et de mono- et di-glycérides, la triacétine, les tensioactifs sarcosinates, les tensioactifs iséthionates, les tensioactifs tautates, les tensioactifs Pluronic, les condensats d'alkylphénols polyoxyéthylénés, les produits dérivés de la condensation d'oxyde d'éthylène avec le produit de la réaction d'oxyde de propylène et d'éthylènediamine, les condensats d'alcools aliphatiques oxyéthylénés, les oxydes d'amines tertiaires à longue chaîne, les oxydes de phosphines tertiaires à longue chaîne, les dialkylsulfoxydes à longue chaîne, et leurs combinaison.

18. Composition de gomme à mâcher selon la revendication 16, dans laquelle ledit agent antibactérien est choisi parmi la chlorhexidine, l'alexidine, les sels d'ammonium quaternaire, le chlorure de benzéthonium, le chlorure de cétylpyridinium, le 2,4,4'-trichloro-2'-hydroxy-diphényléther (triclosan), et leurs combinaisons.

19. Composition de gomme à mâcher selon la revendication 16, dans laquelle ledit composé fluorure est choisi dans le groupe constitué par : le fluorure de sodium, le monofluorophosphate de sodium, le fluorure stanneux et leurs combinaisons.

20. Composition de gomme à mâcher selon la revendication 16, dans laquelle ledit agent antitartre est choisi dans le groupe constitué par : les pyrophosphates, les triphosphates, les polyphosphates, les polyphosphonates, les sels pyrophosphates de di-métal alcalin, les sels polyphosphates de tétra-métal alcalin, le pyrophosphate tétrasodique, le pyrophosphate tétrapotassique, le tripolyphosphate de sodium, et leurs combinaisons.

21. Composition de gomme à mâcher selon la revendication 16, dans laquelle ledit agent antimicrobien est choisi dans le groupe constitué par : le chlorure de cétylpyridinium, les composés du zinc, les composés du cuivre, et leurs combinaisons.

22. Composition de gomme à mâcher selon la revendication 1, dans laquelle ladite couche de gomme sous forme de comprimé comprend en outre un deuxième système Effervescent.

23. Composition de gomme à mâcher selon la revendication 1, dans laquelle ladite couche de gomme sous forme de comprimé comprend en outre un agent de saveur.

24. Composition de gomme à mâcher selon la revendication 1, comprenant en outre un ou plusieurs composants choisis parmi les agents édulcorants, les agents de saveur, les agents colorants, les agents rafraichissants physiologiques, les agents chauffants, et leurs combinaisons.

25. Composition de gomme à mâcher selon la revendication 1, dans laquelle ladite base de gomme à mâcher particulaire est présente en des quantités de 10 % à 80 % en poids de ladite composition de gomme à mâcher.

26. Composition de gomme à mâcher selon la revendication 1, dans laquelle ledit système effervescent est présent en des quantités de 0,5 % à 80 % en poids de ladite composition de gomme à mâcher.

27. Composition de gomme à mâcher selon la revendication 1, comprenant en outre une couche d'enrobage entourant ladite composition de gomme à mâcher.

28. Composition de gomme à mâcher selon la revendication 1, laquelle composition a une teneur totale en eau supérieure à 0 % à 5 % en poids de ladite composition de gomme à mâcher.

29. Composition de gomme à mâcher selon la revendication 1, laquelle composition a une masse volumique de 0,2 à 1,3 g/cm³.

30. Composition de gomme à mâcher selon la revendication 1, laquelle composition a une vitesse de dissolution comprise entre 1 et 20 minutes.
